# EUROPEAN PATENT APPLICATION

(11) **EP 1 878 510 A1**
(43) Date of publication of application: **16.01.2008**
(21) Application number: 06746317.4
(22) Date of filing: 27.04.2006
(51) Int. Cl.: B05D 7/24, B05C 5/02, B05D 1/26, B05D 7/02, D06H 3/00

(54) **METHOD AND DEVICE FOR APPLYING ADHESIVE AGENT TO COATING ELASTIC STRING-LIKE MATERIAL**

(30) Priority: 02.05.2005 JP 2005134199
(71) Applicant: SUN TOOL CORPORATION, Osaka-shi, Osaka 533-0005 (JP); Opelontex Co., Ltd., Tokyo 103-0023 (JP)
(72) Inventor: HIDAKA, Shoji, Sun Tool Corporation, Osaka-shi, Osaka 533-0005 (JP); TARUMI, Seiki, Sun Tool Corporation, Osaka-shi, Osaka 533-0005 (JP); KIDA, Takashi, Sun Tool Corporation, Osaka-shi, Osaka 533-0005 (JP); YAMAZUMI, Eiji, Sun Tool Corporation, Osaka-shi, Osaka 533-0005 (JP); TAKEUCHI, Fumio, Opelontex Co., Ltd., Osaka-shi, Osaka 530-8222 (JP); MARUYAMA, Tetsuo, Opelontex Co., Ltd., Osaka-shi, Osaka 530-8222 (JP); TAKAHIRA, Kenji, Opelontex Co., Ltd., Otsu-shi, Shiga 520-8558 (JP); SHIBATA, Takatoshi, Opelontex Co., Ltd., Otsu-shi, Shiga 520-8558 (JP)
(74) Representative: Knowles, James Atherton
(86) International application number: PCT/JP2006/309520
(87) International publication number: WO 2006/118355

(57) **Abstract**

A method and a device for applying an adhesive to an elastic string-like material capable of performing continuous application operation without stopping the application operation by automatically jumping the knot of the elastic string-like material form a running line for the material at a slit groove in a coater applying head.

The knot of the elastic string-like material running on the coater applying head is raised from the original running line so that it can pass while avoiding the slit groove.

After the knot avoids the slit groove, it is returned to the Original running line and runs continuously. The device comprises a detection means for the knot of elastic string-like material in running and jump guide means for passing the knot while avoiding the slit groove in the coater applying head.

## Description

### TECHNICAL FIELD

The present invention relates to a method and a device for applying an adhesive agent to an elastic string-like material (polyurethane elastic string, flat rubber, and string rubber or the like) on a coating line [such as a manufacturing line of a paper diaper including the step of automatically bonding the string rubber to the diaper body] including a coating step of applying the adhesive agent to the elastic string-like material (polyurethane elastic string, flat rubber, and string rubber or the like). Particularly, the present invention relates to a coating step of applying the adhesive agent to a required elastic string-material (polyurethane elastic string, flat rubber, and string rubber or the like), in a manufacturing apparatus of a body fluid absorbent product (a disposable diaper or the like) including the step of gluing the elastic string-like material (polyurethane elastic string, flat rubber, and string rubber or the like).

### BACKGROUND ART

Regarding the coating step of applying the adhesive agent to the aforementioned elastic string-like material (polyurethane elastic string, flat rubber, and string rubber or the like), an applicant of the present invention (SUNTOOL CORPORATION) discloses the method for applying the adhesive agent to the elastic string-like material (flat rubber, and string rubber or the like) in Japanese Patent Laid Open Publication No.2001-347209 (Patent document 1). According to the patent document 1, the adhesive agent is supplied to a slit groove having an open lower face and an expanded middle part, which communicates with an adhesive supply hole; a tense elastic string-like material (flat rubber, string rubber or the like) is made to horizontally penetrate the expanded part of the aforementioned slit groove; and this elastic string-like material (flat rubber, string rubber or the like) is supplied in a coating line proceeding direction, so that the adhesive agent is applied on an entire circumferential surface of the elastic string-like material passing through the slit groove, on the coating line including the step of applying the adhesive agent to a running elastic string-like body (flat rubber, string rubber or the like).

In the elastic string-like material (polyurethane, flat rubber, string rubber or the like), there is a knot of a terminating end and a starting end of before and behind a supplying bobbin, when replacing the supplying bobbin.

In the aforementioned prior invention, the adhesive agent is applied on the entire circumferential surface of the elastic string-like material (polyurethane elastic string, flat rubber, string rubber or the like) passing through the slit groove. Therefore, there is a problem that a diameter (sectional area) of the elastic string-like material is restricted to an allowable range, according to a relationship with a width (sectional area) of the slit groove. That is, the knot of the elastic string-like material is larger than the width (sectional area) of the slit groove, thus making it impossible to pass through the slit groove.

Accordingly, at the end of the elastic string-like material of the supplying bobbin, an application operation is stopped once to make a knot, and by a manual work, the knot is made to jump at a place of the slit groove, so that the knot of the elastic string-like material is prevented from passing through the slit groove. Therefore, a problem is involved, such as deteriorating work efficiency by stopping a work.

Therefore, an obj ect of the present invention is to enable a continuous application operation to be performed without stopping the application operation, by automatically jumping the knot of the elastic string-like material from a running line of the elastic string-like material at the slit groove.

### DISCLOSURE OF THE INVENTION

A first aspect of the present invention provides a method for applying an adhesive agent to an elastic string-like material, in which the knot of the elastic string-like material can jump the slit groove of a slit nozzle coating part, the method including: detecting a knot that exists in an elastic string-like material running on a coater applying head; based on a detection of the knot, raising the knot from an original running line passing through the slit groove of the slit nozzle coating part so that it can pass while avoiding (jumping) a slit groove; and after the knot avoids the slit groove, returning it to the original running line to run continuously.

A second aspect of the present invention provides a device for applying an adhesive agent to an elastic string-like material, including; detecting means for detecting a knot that exists in a running elastic string-like material; and jump guide means for making the knot pass while avoiding the slit groove by raising the knot from an original running line passing through the slit groove of the coater applying head (slit nozzle coating part), in front of a slit groove of the coater applying head, and after the knot avoids the slit groove, returning it to the original running line to run continuously, so that the knot of the elastic string-like material jumps the slit groove of the coater applying head.

As an embodiment of the first aspect of the present invention, the knot that exists in the running elastic string-like material is detected by being brought into contact with a slide guide body having a detection guide groove 211.

When the knot moves along a front inclined part of the slide guide body, the knot of the elastic string-like material jumps the slit groove of a slit plate. According to an embodiment of the second aspect of the present invention, the knot can smoothly jump and pass through the slit groove by being guided to a front surface of the slit plate.

As an embodiment of the second aspect of the present invention, a slide guide body 210 is provided at a front part in a transfer direction of a head body 207 of a coater applying head 200 so as to oppose to a tip part of the slit groove of a distribution plate 1B in the transfer direction, having the detection guide groove 211 with a width of allowing the elastic string-like material to pass through but inhibiting the knot from passing through, and also the detection guide groove 211 is formed at a position opposite to a slit 202 of the slit plate (distribution plate) in the slide guide body 210, having a width of allowing the elastic string-like material to pass through but inhibiting the knot from passing through, and a second expanded part 205 is formed at a part protruded from the tip end of the slide guide body on the tip part of the slit groove of the slit plate (distribution plate) so as to allow the knot to pass through.

### BRIEF DESCRIPTION OF THE DRAWINGS

Fig. 1 is a perspective view of a coater applying head according to a prior invention.
Fig. 2 is a vertical sectional view of the same.
Fig. 3 is a front view of a coating line for manufacturing a paper diaper.
Fig. 4 is a plan view of the same.
Fig. 5A and 5B are front views of the coater applying head on a manufacturing line of the paper diaper, Fig. 5A shows a case that the coater applying head is applied to a coating step of applying an adhesive agent to a plurality of string rubbers, and Fig. 5B shows a case that the coater applying head is applied to the coating step of applying the adhesive agent to a single flat rubber.
Fig. 6 is a perspective view of the paper diaper.
Fig. 7 is an explanatory view of a first distribution mechanism.
Fig. 8 is an explanatory view of a second distribution mechanism.
Fig. 9 is a sectional view taken along the line S1-S1 of Fig. 8.
Fig. 10 is a sectional view taken along the line S2-S2 of Fig. 8.
Fig. 11 is an explanatory view of a flow of the adhesive agent during an application operation.
Fig. 12 is an explanatory view of a flow of the adhesive agent by the first distribution mechanism and the second distribution mechanism during the application operation.
Fig. 13 is an explanatory view of a flow of the adhesive agent showing a valve action of an extrusion type for an intermittent application.
Fig. 14 is an explanatory view of a flow of the adhesive showing the valve action of a suction type for the intermittent application.
Fig. 15 is a vertical sectional view similar to Fig. 2 of the coater applying head, being an essential part of a device for applying the adhesive agent, according to an example of the prior invention.
Fig. 16 is a front view of a slit plate.
Fig. 17A and 17B are expanded views of a slit groove, showing an example of the elastic string-like material as a string rubber of a rectangular sectional shape.
Fig. 18 is a front view of the coater applying head, being the essential part of the device for applying the adhesive agent, according to an example of the present invention.
Fig. 19 is a vertical sectional view of the same.
Fig. 20 is a left side view of the same.
Fig. 21 is a right side view of the same.
Fig. 22 is a front view of the slit plate.
Fig. 23 is an explanatory view of a numerical value of the slit groove.
Fig. 24A and 24B are explanatory views of a detecting action and a jumping action of the knot of the present invention, Fig. 24A is a vertical sectional view of the coater applying head, and Fig. 24B is a left side view of the same, showing an approach state of the knot to the coater applying head.
Fig. 25A and 25B are explanatory views of the same, showing a jump middle position of the knot.
Fig. 26A and 26B are explanatory views of the same, showing a jump end position of the knot.
Fig. 27A and 27B are explanatory views of the same, showing a landing position of the knot on a running line.
Fig. 28 is an explanatory view of a detecting action and a jumping action of the knot of the present invention, showing a first half step.
Fig. 29 is an explanatory of the same, showing a latter half step.
Fig. 30 is a schematic view showing an outline of the coater applying head, being the essential part of the device for applying adhesive agent, according to a second example of the present invention.
Fig. 31 is a schematic view of the same, showing a jumping state.
Fig. 32A and 32B are schematic views showing a jumping means, Fig. 32A shows the same at a normal time, and Fig. 32B shows the jumping state.

### BEST MODE FOR CARRYING OUT THE INVENTION

Prior to an explanation of an example of the present invention, a device for applying adhesive agent to an elastic string-like material (polyurethane elastic string, flat rubber, string rubber, or the like) on a coating line will be explained with reference to Fig. 1 to Fig. 4.

Fig. 2 and Fig. 3 are views showing an example of using a manufacturing line of a paper diaper including a step of automatically bonding a string rubber to the diaper body, as a coating line including a coating step of applying an adhesive agent H to the elastic string-like material (polyurethane elastic string, flat rubber, string rubber or the like).

A string rubber A or the like, running in a tense state by a tension roller 23 is supplied to an upper part of a band shaped diaper body 22, with an adhesive applied position "e" of the string rubber A running in an upper part of the band shaped diaper body 22 aligned with an elastic material forming place (bonding place of the string rubber or the like) E in the band shaped diaper body 22 which is being transferred on an upper surface of a transfer conveyor 21 in a manufacturing line B.

The band shaped diaper body 22 is cut after a pressurizing step F and a cutting step G, and only the elastic material forming place E of the string rubber Aor the like, is bonded to the band shaped diaper body 22, thus forming an elastic material K, and a "paper diaper" 20 is completed. In the paper diaper shown in Fig. 6, R shows a vertically standing wall by the elastic material K formed of the string rubber.

The prior invention is executed in the step of applying the adhesive agent to the string rubberor the like, for supplying the elastic string-like material (flat rubber, string rubber or the like) A to which the adhesive agent H is intermittently applied in the aforementioned coating line B. The coater applying head B supplies the adhesive agent H to the slit groove having front face, rear face, and bottom face openings in a coating line proceeding direction, to make the string rubber A penetrate the slit groove 10 horizontally in a tense state.

Fig. 5A shows a case of providing a plurality of rubber running slit grooves 10, and the adhesive agent H is applied to the plurality of string rubbers A or the like. Based on a position of such a plurality of rubber running slit grooves 10, mutual intervals P1 and P2 of the string rubbers A, and a positional relation P3 between the slit groove and a base material (band shaped diaper body 22) are restricted, thus making the slit groove 10 function as a guide.

Fig. 5B shows a case of applying the adhesive H to a single string rubber A or the like, by providing only one slit groove 10. Based on the position of the slit groove 10, the positional relations P11 and P12 between the slit groove and the base material (band shaped diaper body 22) are restricted, thus making the slit groove 10 function as a guide.

Fig. 4 shows an outline of the device B for applying adhesive agent to the elastic string-like material (polyurethane elastic string, flat rubber, string rubber or the like) A (hereinafter referred to as string rubber or the like), and reference numeral 1 denotes the coater applying head, reference numeral 2 denotes a valve mechanism, and reference numeral 3 denotes a hot-melt supply source. It is so constituted that the slit groove 10 is vertically formed in a lower end part of the coater applying head 1, a member to be bonded such as a string rubber A is made to horizontally penetrate the expanded part P, and the adhesive agent H is applied to the string rubber Aor the like that passes through the slit groove 10.

With reference to Fig. 4 to Fig. 6, the coater applying head 1 includes a head body 1A, a distribution plate 1B, and a pressing plate 1C.

Fig. 4 shows the head body 1A in which an adhesive supply hole 11 is formed at its upper part so as to communicate with an adhesive agent hole 20 of the valve mechanism 2, a first distribution mechanism 12 having an adhesive horizontal supply passage 13 is formed at its middle part, and a plurality of rubber running slit grooves 10 are vertically formed at a lower end part.

Fig. 7 shows the first distribution mechanism 12 in which an upper adhesive horizontal supply passage 15 and two adhesive longitudinal supply passages 14 (a plurality of adhesive longitudinal supply passages if desired) that communicates between the upper adhesive horizontal supply passage 15 and the adhesive horizontal supply passage 13, are added to form a loop structure, so that the adhesive agent H is supplied from both ends (both ends and a middle) of the adhesive horizontal supply passage 13. Note that the upper adhesive horizontal supply passage 15 can be omitted and only one adhesive longitudinal supply passage 14 may be provided, so as to communicate between the adhesive supply hole 11 and a central part of the adhesive horizontal supply passage 13.

Fig. 8 to Fig. 10 show the distribution plate 1B that forms a second distribution mechanism 16 including a plurality of longitudinal branch flow passages 17 disposed opposed to the adhesive horizontal supply passage 13 of the first distribution mechanism 12 of the coater applying head body 1A; and a distribution reservoir 18 that continues to the lower ends of the longitudinal branch flow passages 17 and disposed opposed to the rubber running slit groove 10 of the coater applying head body 1A.

Fig. 11 shows a case of applying the adhesive agent to the string rubber or the like, by the method for applying adhesive agent to the string rubber or the like, in which a hot-melt is supplied from the hot-melt supply source 3 by off-and-on operation of the valve mechanism 2, to the adhesive supply hole 11 and the first distribution mechanism 12 through the valve mechanism 2, and to a plurality of rubber running slit grooves 10 having front face, rear face, and bottom face openings through the first distribution mechanism 12 and the second distribution mechanism 16.

Depending on an operation type of the valve mechanism, an intermittent coating is possible by an intercept by a counteraction shown in Fig. 15 and by an intercept by a sucking action shown in Fig. 16.

The flow of the hot-melt in the adhesive supply hole 11, the first distribution mechanism 12, and the second distribution mechanism 16, in the coater applying head 1 is shown in Fig. 12.

In addition to the supply from the upper surface of the rubber running slit groove 10, the supply from a position closer to at least one side surface of the rubber running slit groove is added, to thereby apply the adhesive agent H to at least two surfaces of the string rubber or the like, that passes through the rubber running slit groove.

In addition to the supply from the upper surface of the rubber running slit groove 10, the supply from both side surfaces of the rubber running slit groove 10 is added, to thereby apply the adhesive agent H to three surfaces of the string rubber A or the like, that passes through the rubber running slit groove 10.

Next, the coater applying head 1 according to an example of the prior invention will be explained with reference to Fig. 15 to Fig. 19.

In the aforementioned coater applying head 1, the adhesive agent H is applied to two surfaces or three surfaces of the string rubber or the like. Meanwhile, according to the prior invention, the adhesive agent H is applied to an entire circumferential surface of the elastic string-like material (polyurethane elastic string, flat rubber, string rubber or the like).

Fig. 15 and Fig. 16 show a coater applying head 100, being the essential part of the device for applying adhesive agent in the prior invention, in which an expanded part (adhesive reservoir) 103 is formed in an intermediate part of a plurality of vertically arranged slit grooves 102 formed on a slit plate (distribution plate 1B) 101, and the elastic string-like material (polyurethane elastic string, flat rubber, string-like rubber or the like) is passed through the expanded part (adhesive reservoir) 103 filled with the adhesive agent H.

The slit groove 102 is divided into a tip part 102a at a lower side and a supply part 102b at an upper side of the expanded part (adhesive reservoir) 103, and the tip end of the supply part 102b is communicated with an adhesive supply opening 104.

The adhesive supply opening 104 of an upper end of the plurality of slit grooves 102 is communicated with an adhesive hole 105, through a communication groove (distribution groove in a lateral direction) 106. That is, the adhesive agent H supplied to the adhesive hole 106 is supplied to the adhesive supply opening 104 of each slit groove 102 through the communication groove (distribution groove in a lateral direction) 105, and the supply part 102b and the expanded part (adhesive reservoir) 103 are filled with the adhesive agent H.

As the distribution mechanism for supplying the adhesive agent H uniformly to the aforementioned plurality of slit grooves 102, the first distribution mechanism 12 and the second distribution mechanism 16 shown in Fig. 7, Fig. 8, Fig. 12 of the prior invention can be used.

In Fig. 15, reference numeral 107 denotes a head body, and reference numeral 108 denotes a pressing plate (blind plate).

By making the elastic string-like material (polyurethane elastic string, flat rubber, string-like rubber or the like) penetrate the expanded part (adhesive reservoir) 103 of each slit groove 102, the adhesive agent H is applied on an entire circumferential surface of the elastic string-like material (polyurethane elastic string, flat rubber, string rubber or the like).

Fig. 17 shows an expanded slit groove 102, Fig. 17A shows a state that a string-like material A1, whose cross section is circular, penetrates this expanded part and Fig. 17B shows a state that the string-like material A1, whose cross section is rectangular, penetrates this expanded part.

In this way, the adhesive agent H is applied on the entire circumferential surface of the elastic string-like material (polyurethane elastic string, flat rubber, string rubber or the like) A that penetrates the expanded part (adhesive reservoir) 103 of the slit groove 102 having the above-described structure and running in a direction shown by arrow.

List of numerical values according to the prior invention is as follows.
Coating amount of the adhesive agent H 0.03 to 0.06 g/m (per one elastic string-like material)
String-like material A round shaped string rubber 470 dtex
A square shaped string rubber 470 dtex Running speed 150 m/mine
Minimum coating pitch (in case of a plurality of elastic string-like materials) 1.5 mm

Next, explanation will be given to a first example of the present invention with reference to Fig. 18 to Fig. 22.

In a coater applying head 200, a slide guide body 210, which functions as a detecting means and a jump guide means, is positioned at an proceeding direction side of the elastic string-like material (polyurethane elastic string, flat rubber, string rubber or the like) A and is formed as a jump directional extending part of a pressing plate (blind plate) 208.

The slide guide body 210 has a slide face 212 inclined toward the tip end from the running line in side-view, and a detection guide groove 211 is formed in a jumping direction, so as to opposite to a tip part 202a of a slit groove 202.

A width of the detection guide groove 211 is set larger than a sectional face of the elastic string-like material (polyurethane elastic string, flat rubber, string rubber or the like) A, to allow the elastic string-like material (polyurethane elastic string, flat rubber, string rubber or the like) A to pass through freely. However, the width of the detection guide groove 211 is set smaller than the sectional face of a knot P to lock the knot P therein and inhibit a direct advance of the knot P, thus guiding the knot P to a jump position of the tip part of the slide guide body 210.

A second expanded part 205 is formed at the tip end of the slit groove 202 of the slit plate 201, to allow the knot P to pass through.

Regarding the head body 207, the head body 107 of the prior invention is formed to have an opening at a position closer to a jump position from a penetrating position of the elastic string-like material (polyurethane elastic string, flat rubber, string rubber or the like) A, so that the elastic string-like material (polyurethane elastic string, flat rubber, string rubber or the like) A after jump can be landed on an coating line.

Fig. 23 shows a numerical value of each part in the first example.

String rubbers of 470 dtex to 520 dtex will be given as an example. Groove L1 to allow the string rubber to pass through = 0.4 mm Groove L2 to allow the knot to pass through = 1.0 mm Depth L3 = 1 to 3 mm

A groove width of the slit groove is different in proportion to a thickness (dtex) of the string rubber. Note that the coating amount of the adhesive agent H, the string-like material, a running speed minimum coating pitch (in case of a plurality of elastic string-like materials) have the same numerical values as the numerical example of the prior invention.

The aforementioned numerical values are shown as reference examples for understanding the present invention, and various embodiments can be selected. That is, the embodiment can be suitably selected by a publicly-known art and know-how adapted to the characteristic of the adhesive agent H, an obj ect base material to be bonded (a material of a top sheet of a paper diaper or a shape of the paper diaper or the like) and property and shape of the string-like material.

Next, the application operation according to the present invention will be explained.
(a) The knot P approaches the coater applying head 200 (shown in Fig. 24).
(b) The knot P comes in contact with a base part of the slide guide body 210 (shown by solid line in Fig. 28).
(c) The knot P is guided by the detection guide groove 212 along a slide face 211 of the slide guide body 210, and is moved in a jumping direction. (shown by dotted line in Fig. 25 and Fig. 28).
(d) The knot P reaches the tip end of the slide guide body 210 and passes through the second expanded part 205 of the slit groove 202 (shown by one dot chain line in Fig. 26 and Fig. 28).
(e) The knot P overleaps the slit plate 201, and drops toward a guide face 207a of the head body 207 (shown by one dot chain line in Fig. 29).
(f) The knot P lands on the guide face 207a of the head body 207 (shown by solid line in Fig. 27 and Fig. 29).

Thus, the knot P overleaps and avoids the tip part 202a of the slit groove 202 of the slit plate 201 by jumping and passes through the slit groove 202.

In starting the aforementioned application operation, when the knot P that approaches the coater applying head 200, comes in contact with the base part of the slide guide body 210, an advancement of the knot P receives a resistance, and therefore the knot P is guided to be automatically positioned on the front face of the slit groove 202 (note that in the aforementioned state of (a) in Fig. 24, the knot P approaches the coater applying head 200 in a downward (vertical) state to the string rubber, etc. However, the advancement of the knot P receives the resistance even when the knot P is not set in a downward sate (vertical state) but set in a tilted state in right or left, in a lateral state (horizontal state), and in an upward state (vertical state), thus generating a torsion in the string rubber, etc, in the middle of passing through the coater applying head 200. Therefore, the knot P is guided to be automatically positioned on the front face of the slit groove 202).

As a result, when the knot P reaches the state of the aforementioned (d), the knot P is guided to be positioned at the tip end of the slide guide body 210 without fail, thus making it possible to pass through the second expanded part 205 of the slit groove 202 without trouble.

In the above-described embodiment, there exists a section where no adhesive agent is applied, in the before and behind the knot P. However, the rubber string expands and shrinks from an extension state to a natural sate, to change the length of the section where no adhesive agent applies. Therefore, this length can be substantially ignored, having no adverse effect on a bonding action between the base material and the string rubber. This contributes to surely and excellently bonding a plurality of approaching string rubbers, etc, to the base material.

According to the above-described example, in the coater applying head 200 that performs coating action on a lower face, a jumping system, with the head body 207 directed downward, is provided. However, according to the present invention, in case of the coater applying head 200 that performs coating action on a lateral face and upper face, the knot jumps laterally or upward.

A second example of the present invention will be explained with reference to Fig. 30 to Fig. 32.

In the aforementioned example, the slide guide body 210, which functions as detecting means and jump guide means, is provided. The slide guide body 210 functions as detecting means in such a way that the elastic string-like material (polyurethane elastic string, flat rubber, and string rubber or the like) A is moved along the slide face 212 having the detection guide groove 211 in a contact state to lock the knot P into the detection guide groove 211, thereby detecting the knot P, and also functions as jump guide means in such a way that the knot is guided to a jump position at the tip end of the slide guide body 210, with detecting means and jump guide means formed in a single member. However, detecting means 221 and jumping means 222, 222 are separately installed.

As the detecting means 221, each kind of publicly-known sensor mechanism (contact type or non-contact type) such as pressure change sensor type, photoelectric type, and electrostatic capacitance change detection type can be applied.

By a remote control type automatic drive of a pneumatic expansion mechanism and an electric motor-driven type mechanism, or the like, the jumping means 222 is adapted to jump the knot at a coating position of the coater applying head 200, by performing a sequence control of the position of the guide 202a to the position shown in Fig. 30 and Fig. 31, under a microcomputer control, based on an operation of the detecting means 221.

The present invention has an advantage that the knot of the elastic string-like material running on the coater applying head is detected, and the knot is raised from the original running line passing through the slit groove of a slit nozzle coating part, so that it can pass while avoiding the slit groove, and it is returned to the original running line to run continuously. Therefore, even when replacing the supplying bobbin, the application operation needs not to be stopped, thus improving work efficiency by a continuous work of the application operation.

### INDUSTRIAL APPLICABILITY

The present invention contributes to a development of an industry in which a body fluid absorbent product and other textile processed product are manufactured, when it is applied to a coating step of applying the adhesive agent to a required elastic string-like material (polyurethane elastic string, flat rubber, and string rubber or the like), in a manufacturing apparatus of the body fluid absorbent product (a disposable diaper or the like) including the step of gluing the elastic string-like material (polyurethane elastic string, flat rubber, and string rubber or the like).

## Claims

1. A method for applying an adhesive agent to an elastic string-like material, having a coating line including the step of applying the adhesive agent to a running elastic string-like material by a coater applying head, for supplying the adhesive agent to a slit groove, which communicates with an adhesive supply hole and has an open lower face and an expanded middle part, making a tense elastic string-like material penetrate the expanded part of the slit groove horizontally, and supplying the elastic string-like material in a coating line proceeding direction, so that the adhesive agent is applied on an entire circumferential surface of the string-like material passing through the slit groove, the method comprising:
detecting a knot that exists in the elastic string-like material running on the coater applying head; based on a detection of the knot,
raising the knot from an original running line passing through the slit groove of the coater applying head, so that it passes while avoiding the slit groove; and after the knot avoids the slit groove,
returning the running elastic string-like material to the original running line to run continuously, so that when applying the adhesive agent to the running elastic string-like material in the coater applying head, the knot of the elastic string-like material passes and jumps the slit groove.

2. The method for applying the adhesive agent to the elastic string-like material according to claim 1,
wherein when based on the detection of the knot, the knot is raised from the original running line passing through the slit groove of the coater applying head, so that it passes while avoiding the slit groove, the knot of the elastic string-like material is guided to a jumping direction.

3. The method for applying the adhesive agent to the elastic string-like material according to claim 1 or 2,
wherein a plurality of the elastic string-like materials are prepared, so that the adhesive agent is applied on entire circumferential surfaces of the string-like materials passing through a plurality of slit grooves, respectively.

4. An apparatus for applying an adhesive agent to an elastic string-like material, with a coater applying head having a slit groove which communicates with an adhesive supply hole and has an open lower face and an expanded middle part, for applying an adhesive agent to a running elastic string-like material, the apparatus comprising:
detecting means for detecting a knot; and
jump guide means for raising the knot from an original running line passing through the slit groove of the coater applying head and making the knot pass while avoiding the slit groove, based on a detection of the knot,
so that after the knot of the elastic string-like material avoids the slit groove, it is returned to the original running line to run continuously, and jumps and passes the slit groove of the coater applying head.

5. The apparatus for applying the adhesive agent to the elastic string-like material according to claim 4, comprising
a slide guide body as detecting means and jump guide means, positioned in front of a slit plate so as to opposite to a tip part of the slit groove of the slit plate in a transfer direction, and having a detection guide groove of a width allowing the elastic string-like material to pass through but inhibiting the knot from passing through,
wherein the tip part of the slit groove of the slit plate has a second expanded part, which allows the knot to pass through, is formed at a part protruded from a tip end of the slide guide body, so that the knot after jump can land on the running line promptly.

6. The apparatus for applying the adhesive agent to the elastic string-like material according to claim 4, wherein a plurality of slit grooves are prepared, and an adhesive supply opening for supplying the adhesive agent to each slit groove is communicated with an adhesive hole of a coater applying head, through a communication groove, being a lateral distribution groove, so that the adhesive agent is uniformly supplied to the plurality of slit grooves.
